# EUROPEAN PATENT APPLICATION

(11) **EP 4 357 789 A1**
(43) Date of publication of application: **24.04.2024**
(21) Application number: 22845832.9
(22) Date of filing: 13.07.2022
(51) Int. Cl.: G01N 37/00, G01N 35/00, G01N 35/08

(54) **MICROCHIP, SPECIMEN-TESTING DEVICE, AND SPECIMEN-TESTING METHOD**

(30) Priority: 19.07.2021 JP 2021118744
(71) Applicant: HORIBA, Ltd., Minami-ku Kyoto-shi Kyoto 601-8510 (JP)
(72) Inventor: MATSUDA Yasunori, Kyoto-shi, Kyoto 601-8510 (JP); HIRATA Katsuki, Kyoto-shi, Kyoto 601-8510 (JP)
(74) Representative: Isarpatent
(86) International application number: PCT/JP2022/027569
(87) International publication number: WO 2023/002898

(57) **Abstract**

A microchip (3) has a fluid circuit in it. The fluid circuit includes: a specimen introduction portion (31) into which a specimen is introduced; a component separation portion (32) that, when a centrifugal force in a first direction (D1) occurs in the microchip (3), separates, under the centrifugal force in the first direction (D1), a component contained in the specimen introduced into the specimen introduction portion (31); and a reagent reaction portion (33) that has a carrier member (330) carrying a reagent and that makes part of the component introduced from the component separation portion (32) into the carrier member (330) react with the reagent. When a centrifugal force in a second direction (D2) different from the first direction (D1) occurs in the microchip (3), the component separated in the component separation portion (32) is introduced, under the centrifugal force in the second direction (D2), from the component separation portion (32) into the carrier member (330).

## Description

### Technical Field

The present invention relates to a microchip having a fluid circuit, and also relates to a specimen testing apparatus and a specimen testing method for testing a specimen using a microchip.

### Background Art

According to known technology, a drop of a specimen such as blood is put on an analyzer chip so that a particular component contained in the specimen is reacted with a reagent contained in the analyzer chip and the resulting change in chromaticity is optically detected to determine the quantity of the particular component. An example of such analyzer chips is disclosed in Patent Document 1 identified below.

### Citation List

### Patent Literature

Patent Document 1: Japanese Examined Patent Application Publication No. H6-75067

### Summary of Invention

### Technical Problem

When a drop of a specimen is put on an analyzer chip for quantity determination of a particular component, how the dop of the specimen put on the analyzer chip diffuses varies depending on how it is put there. This tends to result in varying reaction times of the particular component in the specimen with the reagent among different sessions of quantity determination, possibly leading to degraded repeatability of quantity determination results (measurement results) with the particular component.

Devised to solve the problem described above, the present invention is aimed at providing a microchip that offers good repeatability of measurement results with a particular component contained in a specimen, and providing a specimen testing apparatus and a specimen testing method for testing a specimen using such a microchip.

### Solution to Problem

According to one aspect of the present invention, a microchip has a fluid circuit in it, and the fluid circuit includes: a specimen introduction portion into which a specimen is introduced; a component separation portion that, when a centrifugal force in a first direction occurs in the microchip, separates, under the centrifugal force in the first direction, a component contained in the specimen introduced into the specimen introduction portion; and a reagent reaction portion that has a carrier member carrying a reagent and that makes part of the component introduced from the component separation portion into the carrier member react with the reagent. When a centrifugal force in a second direction different from the first direction occurs in the microchip, the component separated in the component separation portion is introduced, under the centrifugal force in the second direction, from the component separation portion into the carrier member.

According to another aspect of the present invention, a specimen testing apparatus includes: a rotation mechanism that turns the microchip described above; and an optical detection unit that shines light on the reagent reaction portion of the microchip to receive light reflected therefrom.

According to yet another aspect of the present invention, a specimen testing method includes: a centrifugal separation step of turning a microchip having a fluid circuit in it about a first axis to produce a centrifugal force in a first direction in the microchip, thereby to centrifugally separate a component contained in the specimen introduced into the microchip; an introduction step of turning the microchip about a second axis located at a different position from the first axis, then stopping the microchip, and then turning the microchip about the first axis to produce a centrifugal force in a second direction in the microchip, thereby to introduce the component separated in the centrifugal separation step into a reagent reaction portion having a carrier member carrying a reagent; and a detection step of detecting the concentration of the reaction product of part of the component introduced into the reagent reaction portion with the reagent.

### Advantageous Effects of Invention

According to the present invention, it is possible to achieve good repeatability of measurement results with a particular component in a specimen.

### Brief Description of Drawings

[Fig. 1] is a perspective view showing an outline of the construction of a specimen testing apparatus according to one embodiment of the present invention.
[Fig. 2] is a sectional view showing the structure of a principal part of the specimen testing apparatus.
[Fig. 3] is a block diagram showing the hardware configuration of the specimen testing apparatus.
[Fig. 4] is a top view of a microchip housed in the specimen testing apparatus.
[Fig. 5] is a bottom view of the microchip.
[Fig. 6] is a sectional view showing an outline of the structure of a carrier member applied to the microchip.
[Fig. 7] is a diagram schematically illustrating an example of the reaction in a first reaction portion in the microchip.
[Fig. 8] is a diagram schematically illustrating an example of the reaction in a second reaction portion in the microchip.
[Fig. 9] is a diagram schematically illustrating an example of the reaction in a third reaction portion in the microchip.
[Fig. 10] is a flow chart showing the sequence of steps in a specimen testing method.

### Description of Embodiments

Illustrative embodiments of the present invention will be described below with reference to the accompanying drawings.

### [ 1. Outline of Specimen Testing Apparatus ]

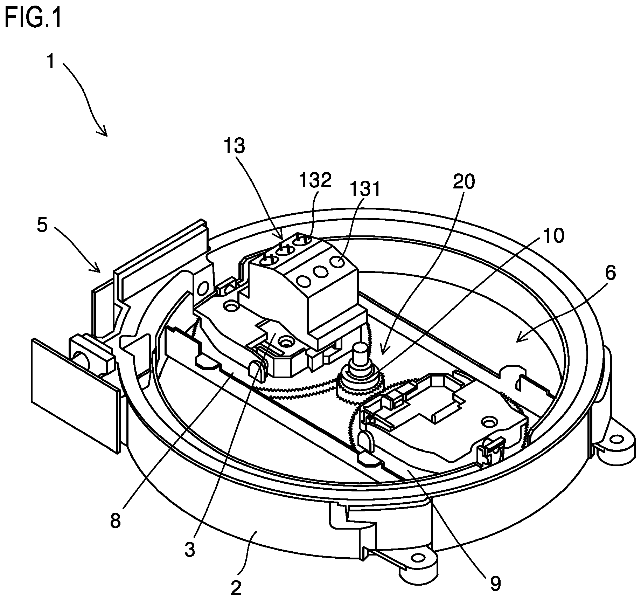

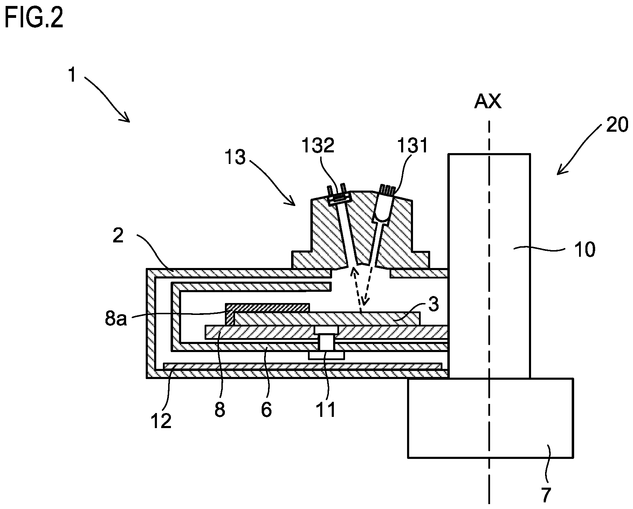

Fig. 1 is a perspective view showing an outline of the construction of a specimen testing apparatus 1 according to an embodiment. Fig. 2 is a sectional view showing the structure of a principal part of the specimen testing apparatus 1. The specimen testing apparatus 1 has a housing 2. Note that, in Fig. 1, to show the construction inside the housing 2, the top face of the housing 2 is omitted from illustration for convenience' sake. The housing 2 has a substantially circular exterior shape as seen from above, though this is not meant to limit its shape.

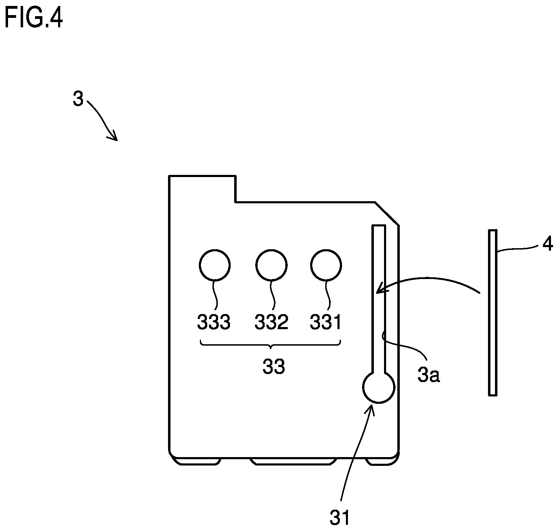

The housing 2 houses a microchip 3 inside it. A specimen stored in a specimen container 4 (see Fig. 4) is introduced into the microchip 3. The specimen is, for example, blood (also called whole blood), blood plasma, or blood serum. The specimen may be any bodily fluid such as saliva, urine, lymph fluid, or cerebrospinal fluid.

The microchip 3 has a micro flow passage inside it. The specimen mentioned above flows through the micro flow passage under the centrifugal force resulting from the microchip 3 turning, and reacts with a reagent previously stored inside the microchip 3. The reaction product of the specimen with the reagent reaches a measurement portion (not illustrated) in the microchip 3. In a side part in the housing 2, a measuring unit 5 is provided, and the light absorbance of the reaction product that has reached the measurement portion of the microchip 3 is optically measured by the measuring unit 5. In this way, based on the result of the measurement of light absorbance as described above, it is possible to calculate the concentration of some components (in the following description also referred to as a "particular component") contained in the specimen.

The optical measurement by the measuring unit 5 is used for measurement of the light absorbance of the reaction product produced by an antigen-antibody reaction of a particular component contained in a specimen with a reagent. Examples of particular components of which the concentration can be calculated by use of an antigen-antibody reaction include Hb (hemoglobin) A1c as an indicator of diabetes, CPR (C-reactive protein) as a maker of inflammation, and CysC (cystatine C) as an indicator used in kidney function testing. Accordingly, different microchips 3 are prepared for HbA1c, CRP, and CysC respectively. A microchip 3 storing a reagent for high-sensitivity detection of CRP (the testing item is called "hsCRP") may be prepared separately from a microchip 3 for ordinary detection of CRP.

In the embodiment, in addition to the optical measurement by the measuring unit 5 as described above, optical measurement by an optical detection unit 13, which will be described later, can also be preformed. The optical measurement by the optical detection unit 13 is used chiefly for measurement of the concentration of the reaction product produced by an enzyme reaction between a particular component contained in a specimen and a reagent. Here, an enzyme reaction between a particular component and a reagent is to be understood to include an enzyme reaction between a component produced by hydrolysis or the like of some components contained in a specimen and a reagent. The microchip 3 as the target of the optical measurement by the optical detection unit 13 as mentioned above will be described in detail later.

In the housing 2, a rotary table 6 is provided. As shown in Fig. 2, the rotary table 6 is turned by a motor 7 about a rotation axis AX. On the rotary table 6, a first stage 8 and a second stage 9 (see Fig. 1) are provided. The first and second stages 8 and 9 are arranged at positions that are point-symmetric about the rotation axis AX as seen along the rotation axis AX.

On the first stage 8, the microchip 3 described above is placed and is fixed on top of the first stage 8 with a holder 8a. On the second stage 9, a balancer chip (dummy chip) for keeping a balance with the microchip 3 is placed and is fixed on top of the second stage 9 with a holder (not illustrated). Another microchip 3 may instead be fixed to the second stage 9.

The first and second stages 8 and 9 are coupled to a driving force switching mechanism 10, which includes a gear and a cam. The driving force switching mechanism 10 switches the transmission of the driving force of the motor 7 to the first and second stages 8 and 9. This switches whether to turn the first and second stages 8 and 9 or not, and switches the direction of the centrifugal force that acts on the microchip 3 as the rotary table 6 turns. By switching the turning of the first and second stages 8 and 9 in this way, it is possible to control the direction in which a specimen flows in the microchip 3.

The first stage 8 turns (rotates about an axis through itself) about a first planetary shaft 11 fitted to the rotary table 6. The first planetary shaft 11 is located away from the rotation axis AX of the rotary table 6 in a radial direction, and is disposed parallel to the rotation axis AX. Accordingly, the first stage 8 can rotate about the first planetary shaft 11 and revolve around the rotation axis AX. Likewise, the second stage 9 turns (rotates about an axis through itself) about a second planetary shaft (not illustrated) fitted to the rotary table 6. The second planetary shaft is located opposite from the first planetary shaft 11 with respect to the rotation axis AX of the rotary table 6, and is disposed parallel to the rotation axis AX. Thus, the second stage 9 can rotate about the second planetary shaft and revolve about the rotation axis AX.

Thus, at least the motor 7, the first stage 8, the second stage 9, the driving force switching mechanism 10, and the first planetary shaft 11 constitute a turning mechanism 20 for turning the microchip 3.

In a bottom part in the housing 2, a heater 12 is fitted. The heater 12 functions as a temperature control unit for controlling the temperature inside the housing 2. By controlling the amount of heat generated by the heater 12, it is possible to keep a constant temperature (e.g., 37°C) inside the housing 2.

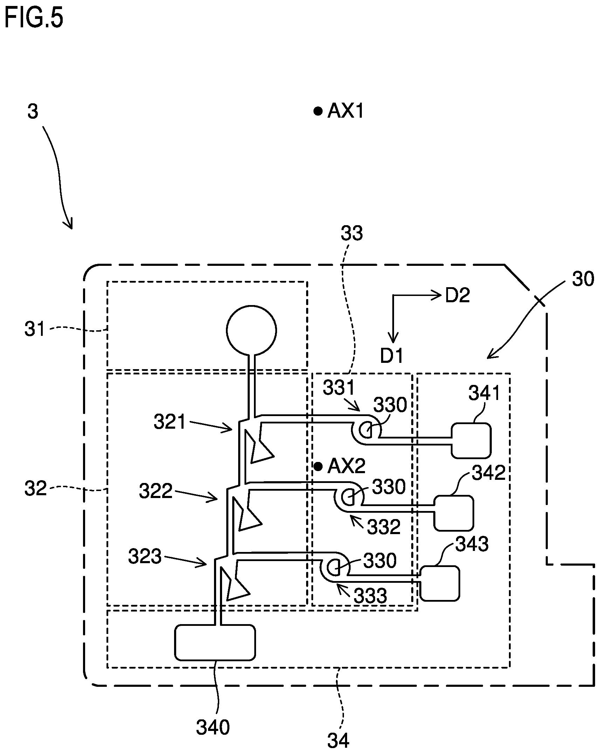

On the top face of the housing 2, an optical detection unit 13 is provided. The optical detection unit 13 includes a light emitter 131 and a light receiver 132. The light emitter 131 comprises, for example, a three-color LED (light-emitting diode) that emits light with wavelengths of R (red), G (green), and B (blue). The light receiver 132 comprises a sensor (e.g., a photodiode) that receives the light emitted from the light emitter 131 and reflected from a reagent reaction portion 33 (see Fig. 5), described later, in the microchip 3, and has sensitivity in wavelength ranges corresponding to RGB. By receiving the above-mentioned reflected light with the light receiver 132, it is possible to detect the concentration of the dye produced by a reaction in the reagent reaction portion 33. It is thus possible to detect the concentration of a particular component contained in a specimen by a colorimetric method.

In the embodiment, the set of the light emitter 131 and the light receiver 132 in the optical detection unit 13 is provided so as to correspond to the number and positions of reaction portions (a first reaction portion, a second reaction portion, ...) constituting the reagent reaction portion 33 for the microchip 3 used. Accordingly, for example, in a configuration that uses a microchip 3 with three reaction portions constituting the reagent reaction portion 33, as shown in Fig. 1, three sets of a light emitter 131 and a light receiver 132 are provided in the optical detection unit 13 so as to correspond to the positions of those reaction portions in the microchip 3. The optical detection unit 13 may include only one set, or two sets, or four or more sets of a light emitter 131 and the light receiver 132.

While in the embodiment the optical detection unit 13 is provided only over the first stage 8 in the housing 2, another optical detection unit 13 may be provided also over the second stage 9 to cope with a microchip 3 that may be fixed to the second stage 9.

### [ 2. Hardware Configuration of Specimen Testing Apparatus ]

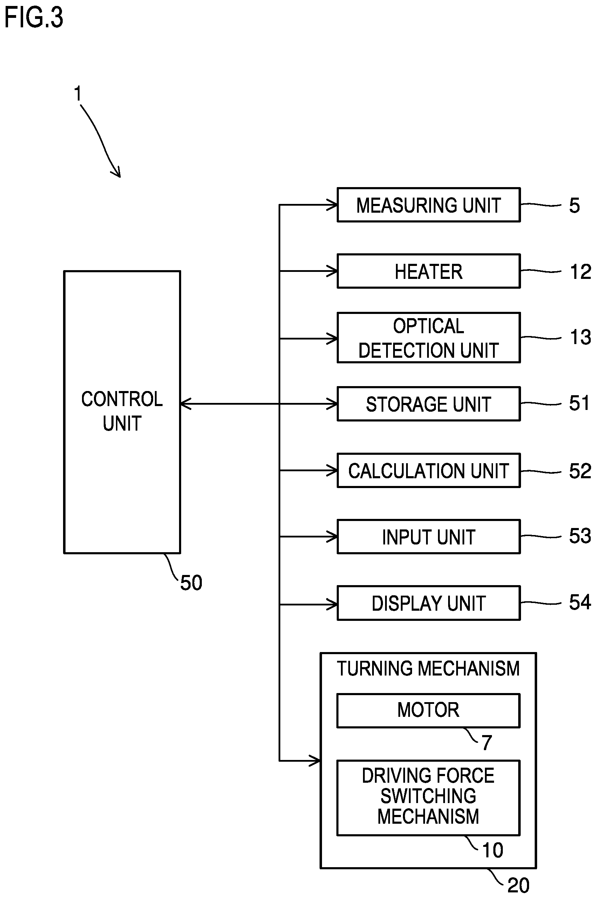

Fig. 3 is a block diagram showing the hardware configuration of the specimen testing apparatus 1. The specimen testing apparatus 1 further includes a control unit 50, a storage unit 51, a calculation unit 52, an input unit 53, and a display unit 54. The control unit 50 comprises, for example, a central arithmetic processing device called a CPU (central processing unit), and controls the operation of different parts of the specimen testing apparatus 1. For example, the control unit 50 controls the operation of the measuring unit 5, the heater 12, the optical detection unit 13, and the turning mechanism 20 described above. The storage unit 51 is a memory that stores operation programs for the control unit 50 as well as different kinds of information, and includes a ROM (read-only memory), a RAM (random-access memory), a nonvolatile memory, and the like.

The calculation unit 52 is fed with the results of the measurement by the measuring unit 5 and the optical detection unit 13, subjects them to predetermined arithmetic processing, and outputs the results as measured values. The calculation unit 52 may be configured as a dedicated arithmetic processing circuit. Or the control unit 50 (CPU) may function also as the calculation unit 52. The input unit 53 comprises buttons, switches, a touch panel, and the like for receiving various instructions from a user. The display unit 54 comprises a display device such as a liquid crystal display device, and displays the measured values output from the calculation unit 52. When the input unit 53 is configured with a touch panel, the touch panel may be provided on the top face of the display unit 54.

### [ 3. Details of Microchip ]

### ( 3-1. Appearance of Microchip )

Next, the microchip 3 as the target of the optical measurement by the optical detection unit 13 will be described in detail. Fig. 4 is a top view of the microchip 3. The microchip 3 has a container compartment 3a. The specimen container 4 is fitted into, so as to be accommodated in, the container compartment 3a. The specimen container 4 is also called a capillary or capillary tube. The specimen container 4 stores a specimen collected from a test subject. It is here assumed that the specimen is whole blood.

For easy fitting of the specimen container 4 into the container compartment 3a, the container compartment 3a is formed in a shape slightly larger than the exterior shape of the specimen container 4. In particular, a lower-end part of the container compartment 3a is formed to have a larger width than the rest of it, in a substantially circular shape as seen in a plan view. The lower-end part of the container compartment 3a constitutes a specimen introduction portion 31 where a specimen is introduced from the specimen container 4 into the microchip 3. Fig. 4 also show the reagent reaction portion 33 (a first reaction portion 331, a second reaction portion 332, and a third reaction portion 333), which will be described in detail later..

### ( 3-2. Fluid Circuit in Microchip )

Fig. 5 is a bottom view of the microchip 3 described above. Note that Fig. 5 shows the microchip 3 as seen from below, turned 90° clockwise compared with the microchip 3 in Fig. 4. As shown in the diagram, the microchip 3 has a fluid circuit 30 inside it. The fluid circuit 30 comprises micro flow passages through which a specimen in the form of a fluid flows. The fluid circuit 30 includes the specimen introduction portion 31 mentioned above, a component separation portion 32, a reagent reaction portion 33, and a waste fluid collection portion 34. The waste fluid collection portion 34 connects to the component separation portion 32 and the reagent reaction portion 33. The waste fluid collection portion 34 includes a specimen waste fluid portion 340, a first waste fluid portion 341, a second waste fluid portion 342, and a third waste fluid portion 343. The waste fluid collection portion 34 will be described in detail later.

### < Component Separation Portion >

The component separation portion 32 separates a component contained in the specimen introduced into the specimen introduction portion 31 under a centrifugal force that occurs in the microchip 3. Setting the microchip 3 in the specimen testing apparatus 1 and turning the microchip 3 with the turning mechanism 20 produces the centrifugal force. In particular, when a centrifugal force occurs in direction D1 (first direction) shown in Fig. 5 with respect to the microchip 3, under this centrifugal force in direction D1, the component separation portion 32 separates a component (e.g., blood plasma) contained in the specimen from the specimen as originally introduced (e.g., whole blood).

Here, a centrifugal force in direction D1 can be produced in the microchip 3 in the following manner. The microchip 3 is first turned about the rotation axis AX2 with the turning mechanism 20 (see Fig. 2) and is then stopped at the position (first position) shown in Fig. 5. That is, when the microchip 3 is located as shown in Fig. 5 with respect to the rotation axes AX1 and AX2, the turning of the microchip 3 about the rotation axis AX2 is stopped. The rotation axis AX2 corresponds to the rotation axis of the first stage 8, that is, the rotation axis of the first planetary shaft 11. The rotation axis AX1 corresponds to the rotation axis AX of the rotary table 6 show in Fig. 2. The microchip 3 is turned about the rotation axis AX1. This causes a centrifugal force in direction D1 to act on the microchip 3.

The component separation portion 32 described above has a plurality of individual separation portions that connect to the specimen introduction portion 31. Specifically, the component separation portion 32 has, as the individual separation portions just mentioned, a first separation portion 321, a second separation portion 322, and a third separation portion 323. The first, second, and third separation portions 321, 322, and 323 are arranged in this order halfway along the flow passage leading from the specimen introduction portion 31 to the waste fluid collection portion 34. The number of individual separation portions is not limited to three; it may be two, or four or more. The component separation portion 32 may have only one individual separation portion.

When under the centrifugal force in direction D1 the specimen is introduced from the specimen introduction portion 31 into the first separation portion 321 in the component separation portion 32, a predetermined amount is measured in the first separation portion 321 so that the predetermined amount of the specimen remains in the first separation portion 321. The rest of the specimen, that is, the specimen that exceeds the just-mentioned predetermined amount, overflows out of the first separation portion 321 to be introduced into the second separation portion 322.

Likewise, in the second separation portion 322, under the centrifugal force in direction D1 a predetermined amount is measured so that only the predetermined amount of the specimen remains in the second separation portion 322. The rest of the specimen, that is, the specimen that exceeds the just-mentioned predetermined amount, overflows out of the second separation portion 322 to be introduced into the third separation portion 323.

Likewise, also in the third separation portion 323, under the centrifugal force in direction D1 a predetermined amount is measured so that only the predetermined amount of the specimen remains in the third separation portion 323. The rest of the specimen, that is, the specimen that exceeds the just-mentioned predetermined amount, overflows out of the third separation portion 323 to be introduced into the waste fluid collection portion 34.

Moreover, in the first, second, and third separation portions 321, 322, and 323, under the centrifugal force in direction D1, the specimen is separated into blood plasma and blood cells. That is, in the first, second, and third separation portions 321, 322, and 323, a component contained in the specimen is separated. In the first, second, and third separation portions 321, 322, and 323, blood plasma collects upstream along direction D1, and blood cells collect downstream along direction D1.

As described above, owing to the component separation portion 32 having a plurality of individual separation portions (the first, second, and third separation portions 321, 322, and 323), the following benefits are obtained. A specimen is first distributed among a plurality of individual separation portions and then centrifugal separation is performed in each individual separation portion; thus centrifugal separation takes less time than if it is performed without the specimen being distributed in a plurality of parts. Moreover, when a component distributed in a plurality of parts is separated in each individual separation portion, the supernatant component (the part that stays at the top as a result of centrifugal separation) can be extracted under the same conditions in each individual separation portion. It is thus possible to obtain satisfactory repeatability. Note that, if a plurality of supernatant components are extracted without a specimen distributed among a plurality of individual separation portions, they are extracted from different positions of the supernatant component obtained in a single separation portion; thus may result in variations in properties among the different supernatant components extracted.

### < Reagent Reaction Portion >

The reagent reaction portion 33 has carrier members 330 that carry a reagent. Under a centrifugal force occurring in the microchip 3, the reagent reaction portion 33 makes part of the component introduced from the component separation portion 32 into a carrier member 330 react with the reagent carried by the carrier member 330.

Here, the direction of the above-mentioned centrifugal force that occurs in the microchip 3 is direction D2 (second direction), which is different from direction D1. A centrifugal force in direction D2 can be produced in the microchip 3 in the following manner. First, the microchip 3 is turned 90° clockwise in Fig. 5 about the rotation axis AX2 shown in Fig. 5 with the turning mechanism 20 (see Fig. 2) and is stopped at that position (second position). Next, the microchip 3 is turned about the rotation axis AX1.

The reagent reaction portion 33 has a plurality of individual reaction portions that connect respectively to the individual separation portions in the component separation portion 32. Specifically, the reagent reaction portion 33 has, as the just-mentioned individual reaction portions, a first reaction portion 331, a second reaction portion 332, and a third reaction portion 333. The first, second, and third reaction portions 331, 332, and 333 connect to the first, second, and third separation portions 321, 322, and 323 respectively. The first, second, and third reaction portions 331, 332, and 333 each has a carrier member 330 as described above.

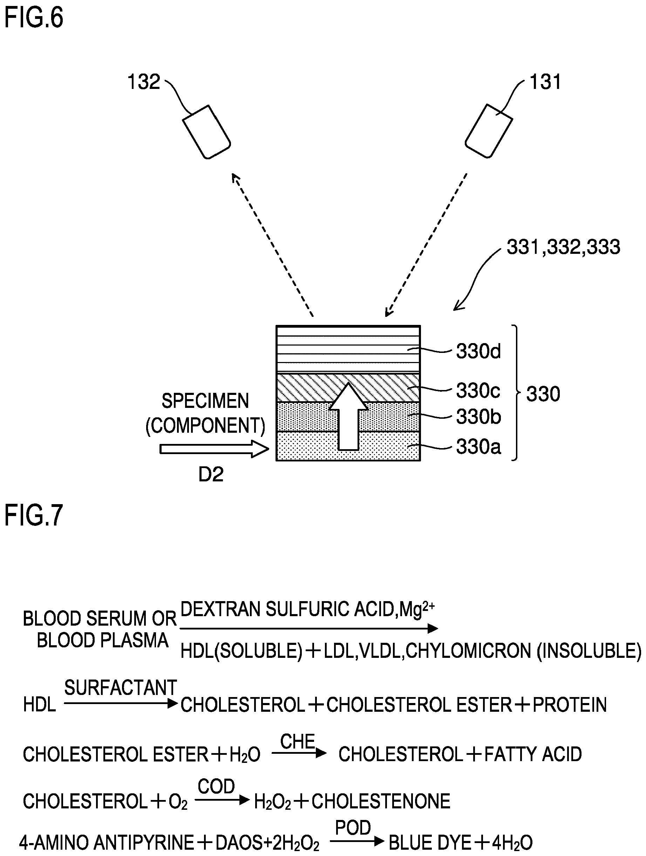

Fig. 6 is a sectional view showing an outline of the structure of the carrier member 330. The carrier member 330 is configured with a development layer 330a, a light shield layer 330b, a reaction layer 330c, and a support layer 330d stacked on each other in this order from bottom up. The development layer 330a, the light shield layer 330b, the reaction layer 330c, and the support layer 330d are all formed of a porous sheet. Usable as the porous sheet is, for example, a fibrous porous sheet such as a nonwoven fabric, or a non-fibrous porous sheet such as a resin sheet. Usable as the resin for the resin sheet is, for example, a cellulose ester resin.

In the carrier member 330, the reagent mentioned above is held in the reaction layer 330c. The reagent is a dry reagent containing an enzyme. The light shield layer 330b contains light-shielding microparticles such as titanium dioxide. The development layer 330a, the light shield layer 330b, and the reaction layer 330c are supported to be flat by the support layer 330d. The development layer 330a may contain a surfactant.

A component (blood plasma) centrifugally separated in the first separation portion 321 in the component separation portion 32 and introduced into the first reaction portion 331 in the reagent reaction portion 33 is introduced into the development layer 330a in the carrier member 330, and is diffused and developed in a direction perpendicular to the thickness direction of the carrier member 330 (i.e., the stack direction of the layers). By capillary action, this component moves up in the development layer 330a and across the light shield layer 330b to permeate the reaction layer 330c. In the reaction layer 330c, through an enzyme reaction of part (particular component) of the component with the reagent, a reaction product (e.g., a blue dye) is produced.

Fig. 7 schematically shows an example of the reaction in the first reaction portion 331. In the diagram, CHE (cholesterol esterase), COD (cholesterol oxidase), and POD (peroxidase) denote enzymes, and the reactions ascribable to those enzymes are enzyme reactions.

Likewise, a component (blood plasma) centrifugally separated in the second separation portion 322 in the component separation portion 32 and introduced into the second reaction portion 332 in the reagent reaction portion 33 is developed in the development layer 330a in the carrier member 330 in the second reaction portion 332 and, by capillary action, moves across the light shield layer 330b to permeate the reaction layer 330c. In the reaction layer 330c, through an enzyme reaction of part (particular component) of the component with the reagent, a reaction product (e.g., a blue dye) is produced.

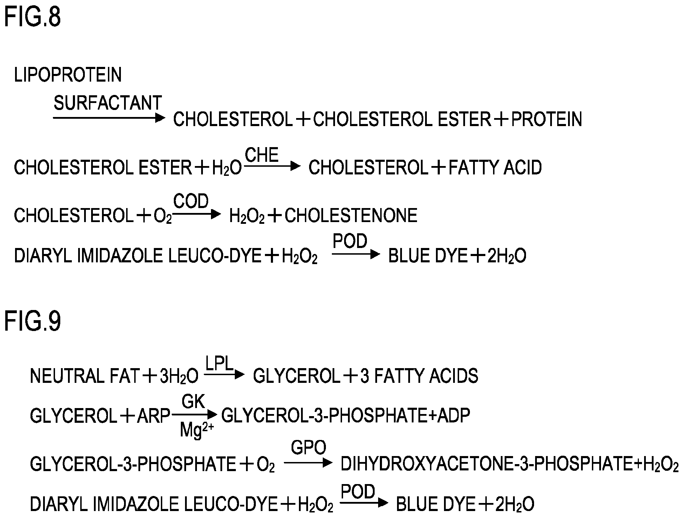

Fig. 8 schematically shows an example of the reaction in the second reaction portion 332. In the diagram, CHE, COD, and POD denote enzymes, and the reactions ascribable to those enzymes are enzyme reactions.

Likewise, a component (blood plasma) centrifugally separated in the third separation portion 323 in the component separation portion 32 and introduced into the third reaction portion 333 in the reagent reaction portion 33 is developed in the development layer 330a in the carrier member 330 in the third reaction portion 333 and, by capillary action, moves across the light shield layer 330b to permeate the reaction layer 330c. In the reaction layer 330c, through an enzyme reaction of part (particular component) of the component with the reagent, a reaction product (e.g., a blue dye) is produced.

Fig. 9 schematically shows an example of the reaction in the third reaction portion 333. In the diagram, LPL (lipoprotein lipase), GK (glycerol kinase), GPO (glycerol-3-phosphate oxidase), and POD denote enzymes, and the reactions ascribable to those enzymes are enzyme reactions.

The light that travels from the light emitter 131 in the optical detection unit 13 to the first reaction portion 331 passes through the support layer 330d in the carrier member 330 in the first reaction portion 331 to strike the reaction layer 330c. By receiving the light reflected from the reaction layer 330c with the light receiver 132, it is possible to obtain a detection result corresponding to the amount of light received. Specifically, it is possible to detect the concentration of the blue dye produced by the reaction in the reaction layer 330c in the first reaction portion 331. Based on this concentration of the blue dye, it is possible to detect the HDL (high-density lipoprotein)-cholesterol concentration in blood plasma.

Likewise, the light that travels from the light emitter 131 in the optical detection unit 13 to the second reaction portion 332 passes through the support layer 330d in the carrier member 330 in the second reaction portion 332 to strike the reaction layer 330c. By receiving the light reflected from the reaction layer 330c with the light receiver 132, it is possible to detect the concentration of the blue dye produced by the reaction in the reaction layer 330c in the second reaction portion 332. Based on this concentration, it is possible to detect the total cholesterol concentration in blood plasma.

Likewise, the light that travels from the light emitter 131 in the optical detection unit 13 to the third reaction portion 333 passes through the support layer 330d in the carrier member 330 in the third reaction portion 333 to strike the reaction layer 330c. By receiving the light reflected from the reaction layer 330c with the light receiver 132, it is possible to detect the concentration of the blue dye produced by the reaction in the reaction layer 330c in the third reaction portion 333. Based on this concentration, it is possible to detect the neutral fat concentration in blood plasma.

In this way, by detecting the concentrations of three particular components in blood plasma simultaneously, it is possible to check the lipid metabolism of a test subject.

### < Waste Fluid Collection Portion >

Next, the waste fluid collection portion 34 shown in Fig. 5 will be described in detail. The specimen waste fluid portion 340 in the waste fluid collection portion 34 connects to the component separation portion 32 (in particular, the third separation portion 323), and is located downstream of the component separation portion 32 along direction D1. Under a centrifugal force in direction D1, the specimen overflowing out of the third separation portion 323 is introduced into the specimen waste fluid portion 340 to be stored there.

The first waste fluid portion 341 connects to the first reaction portion 331 in the reagent reaction portion 33, and is located downstream of the first reaction portion 331 along direction D2. Under a centrifugal force in direction D2, a component of the specimen that has passed through the first reaction portion 331 along direction D2 is introduced into the first waste fluid portion 341 to be stored there.

The second waste fluid portion 342 connects to the second reaction portion 332 in the reagent reaction portion 33, and is located downstream of the second reaction portion 332 along direction D2. Under a centrifugal force in direction D2, a component of the specimen that has passed through the second reaction portion 332 along direction D2 is introduced into the second waste fluid portion 342 to be stored there.

The third waste fluid portion 343 connects to the third reaction portion 333 in the reagent reaction portion 33, and is located downstream of the third reaction portion 333 along direction D2. Under a centrifugal force in direction D2, a component of the specimen that has passed through the third reaction portion 333 along direction D2 is introduced into the third waste fluid portion 343 to be stored there.

### [ 4. Specimen Testing Method ]

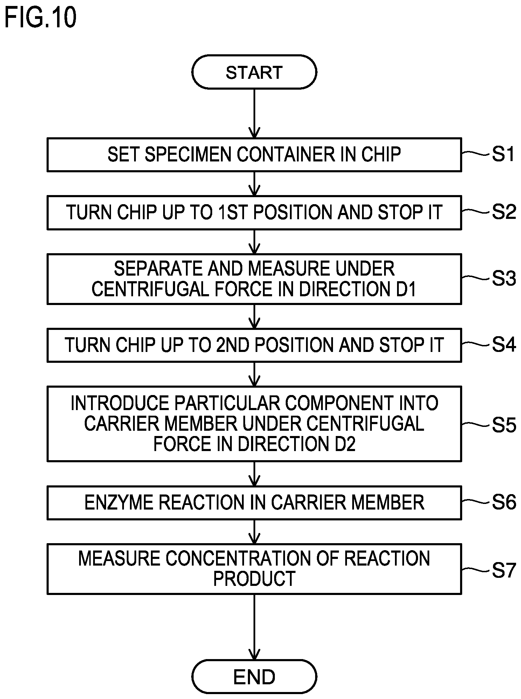

Fig. 10 is a flow chart showing the sequence of steps in a specimen testing method carried out on the specimen testing apparatus 1 according to the embodiment. First, a user (doctor, clinical technician, or the like) sets the specimen container 4 in, by inserting it into, the container compartment 3a in the microchip 3 (S 1). Thus, the specimen in the specimen container 4 is introduced into the specimen introduction portion 31 in the microchip 3. Next, under the control of the control unit 50, the turning mechanism 20 turns the microchip 3 about the rotation axis AX2 and stops the microchip 3 at a first position (the position shown in Fig. 4) (S2).

Subsequently, the turning mechanism 20 turns the microchip 3 about the rotation axis AX1 to produce a centrifugal force in direction D1 in the microchip 3. Thus, the specimen introduced into the specimen introduction portion 31 is fed to the component separation portion 32 (the first, second, and third separation portions 321, 322, and 323) and, in the component separation portion 32, a component contained in the specimen is centrifugally separated (S3: centrifugal separation step). At S3, in addition to centrifugal separation, measurement of a predetermined amount is performed in the component separation portion 32. The specimen exceeding the predetermined amount is fed to the waste fluid collection portion 34 under the centrifugal force in direction D 1.

Next, the turning mechanism 20 turns the microchip 3 about the rotation axis AX2 and stops it at the second position mentioned above. That is, the turning mechanism 20 stops the microchip 3 at the position turned 90° clockwise about the rotation axis AX2 from the position of the microchip 3 shown in Fig. 5 (S4). After that, the turning mechanism 20 turns the microchip 3 about the rotation axis AX1 to produce a centrifugal force in direction D2 in the microchip 3. Thus, the component separated in the centrifugal separation step at S3 is introduced into the reagent reaction portion 33 (the first, second, and third reaction portions 331, 332, and 333) carrying a reagent (S5: introduction step). In the reagent reaction portion 33, part (a particular component) of the introduced component engage in an enzyme reaction with the reagent to produce a reaction product (e.g., blue dye) (S6).

After that, the optical detection unit 13 shines light on the reagent reaction portion 33 and receives the reflected light to detect the concentration (color) of the reaction product (S7: detection step). Based on the detection result at S7, it is possible to determine the quantity of the particular component.

### [ 5. Effects ]

As described above, in the microchip 3 according to the embodiment, when a centrifugal force in a first direction (e.g., direction D1) occurs in the microchip 3, under the centrifugal force in the first direction, the component separation portion 32 separates a component contained in a specimen introduced into the specimen introduction portion 31. The reagent reaction portion 33 makes part (a particular component) of the component introduced from the component separation portion 32 into the carrier member 330 react with a reagent. Here, when a centrifugal force in a second direction (e.g., direction D2) different from the first direction occurs in the microchip 3, under the centrifugal force in the second direction, the above-mentioned component separated in the component separation portion 32 is introduced from the component separation portion 32 into the carrier member 330.

Using the microchip 3 configured as described above, it is possible, while appropriately controlling the direction (first or second direction) of the centrifugal force occurring in the microchip 3, to introduce the specimen introduced into the specimen introduction portion 31 further into the component separation portion 32 to separate a component, and to make the separated component reach the carrier member 330 in the reagent reaction portion 33 to make part (particular component) of that component react with a reagent. It is thus possible to optically (with the optical detection unit 13) detect the color of a reaction product (ex, blue dye) produced in the reagent reaction portion 33 to determine the quantity of the particular component. The direction of the above-mentioned centrifugal force can be easily controlled by controlling the turning mechanism 20 in the specimen testing apparatus 1.

Using the microchip 3, it is possible, by controlling the direction and magnitude of the centrifugal force occurring in the microchip 3, to control and equalize, for each instance of introduction (partial dispensation) of a component from the component separation portion 32 into the reagent reaction portion 33, the speed (partial dispensation speed) and the position (partial dispensation position) at which the component separated in the component separation portion 32 is introduced into the carrier member 330 in the reagent reaction portion 33. It is thus possible to reduce variations in the reaction time of the particular component with the reagent among instances of partial dispensation. It is hence possible to obtain satisfactory repeatability in measurement results with the particular component. That is, it is possible to obtain (approximately) equal quantity determination results for equal amounts of the particular component. The magnitude of the above-mentioned centrifugal force can be easily controlled by controlling the speed at which the microchip 3 is turned by the turning mechanism 20 in the specimen testing apparatus 1.

The first, second, and third separation portions 321, 322, and 323 as a plurality of individual separation portions of the component separation portion 32 each separate and measure, under the centrifugal force in the first direction, a component contained in the specimen introduced into the specimen introduction portion 31.

With this configuration, under the centrifugal force in the first direction occurring in the microchip 3, the component contained in the specimen can be separated and measured simultaneously in a plurality of individual separation portions, that is, at a plurality of places.

The first, second, and third reaction portions 331, 332, and 333 as a plurality of individual reaction portions of the reagent reaction portion 33 each make part of the component introduced from the individual separation portion (the first, second, or third separation portions 321, 322, or 323) into the carrier member 330 under the centrifugal force in the second direction react with the reagent carried by the carrier member 330.

With this configuration, based on the reaction products (e.g., blue dye) produced in the plurality of individual reaction portions of the reagent reaction portion 33, that is, the first, second, and third reaction portions 331, 332, and 333, it is possible to determine the quantities of different particular components simultaneously.

As shown in Fig. 6, the carrier member 330 in the reagent reaction portion 33 includes a development layer 330a and a reaction layer 330c. The development layer 330a diffuses and develops the component introduced from the component separation portion 32. The reaction layer 330c is located above the development layer 330a, and holds a reagent. The reaction layer 330c is penetrated by the component developed in the development layer 330a.

In the carrier member 330 configured as described above, the component developed in the development layer 330a permeates the reaction layer 330c, and part (a particular component) of the component reacts with the reagent in the reaction layer 330c. Thus, by shing light on the carrier member 330 from the reaction layer 330c side, that is, from above the microchip 3, and receiving the light obtained by reflection on the reaction layer 330c, it is possible to determine the quantity of the particular component. Also with a configuration where the reaction layer 330c is located above the development layer 330a, the component developed in the development layer 330a permeates the reaction layer 330c, and thus it is possible to reliably make the reagent and the particular component react with each other in the reaction layer 330c. Permeation of the reaction layer 330c by the component can be achieved easily by use of capillary action with the layers of the carrier member 330 formed of porous sheets as in the embodiment.

The carrier member 330 includes a light shield layer 330b. The light shield layer 330b is located between the development layer 330a and the reaction layer 330c.

In a case where light is shone on the carrier member 330 from the reaction layer 330c side, even if part of the light that strikes the reaction layer 330c is transmitted through the reaction layer 330c, it is intercepted by the light shield layer 330b. It is thus possible to reduce the entry into the development layer 330a, and the reflection from the development layer 330a, of the light transmitted through the reaction layer 330c. If the light transmitted through the reaction layer 330c is reflected from the development layer 330a and mixes with the light reflected from the reaction layer 330c, it is difficult to accurately measure (determine the quantity of) the particular component. By reducing the entry and reflection of light into and from the development layer 330a as described above, it is possible to accurately determine the quantity of the particular component.

The reagent carried by the carrier member 330 (carried in a dry state in the porous sheet forming the reaction layer 330c) is a dry reagent. This reliably makes it possible to obtain a configuration that, while holding the reagent in the reaction layer 330c in the carrier member 330, makes the component developed in the development layer 330a permeate the reaction layer 330c to make the particular component react with the reagent in the reaction layer 330c.

The reagent carried by the carrier member 330 contains an enzyme. This makes it possible to produce a microchip 3 that can determine the quantity of a particular component based on the color of the reaction product produced by an enzyme reaction.

The waste fluid collection portion 34 in the fluid circuit 30 in the microchip 3 is located downstream of the component separation portion 32 along the first direction, and is located downstream of the reagent reaction portion 33 along the second direction.

Under the centrifugal force in the first direction, the specimen overflowing out of the component separation portion 32 (the specimen exceeding a predetermined amount) is diverted to the waste fluid collection portion 34 located downstream; under the centrifugal force in the second direction, the component fed from the component separation portion 32 and having passed through the reagent reaction portion 33 (carrier member 330) in the second direction is diverted to the waste fluid collection portion 34 located downstream; thus they can be stored together in the waste fluid collection portion 34. Thus, after quantity determination of the particular component, the microchip 3 as a whole can be disposed of to achieve hygienic disposal. This eliminates the need for special treatment for sanitizing the waste fluid and the need for special knowledge about the disposal (sanitization) of the waste fluid.

The specimen testing apparatus 1 according to the embodiment includes a turning mechanism 20 for turning a microchip 3 and an optical detection unit 13 for shining light on a reagent reaction portion 33 in the microchip 3 and receiving the reflected light.

With this configuration, it is possible to detect with the optical detection unit 13 the color of the reaction product produced in the reagent reaction portion 33. It is thus possible to determine the amount of a particular component based on the detection result from the optical detection unit 13.

The specimen testing apparatus 1 further includes a measuring unit 5 (see Fig. 1) for measuring the light absorbance of the reaction product. Providing the optical detection unit 13 and the measuring unit 5, which are different measurement systems, on the same apparatus makes it possible to control, for example, temperature under the same conditions. It is thus possible to perform measurement with satisfactory repeatability.

On one hand, the turning mechanism 20 turns the microchip 3 about the rotation axis AX1 (first axis) to produce a centrifugal force in a first direction in the microchip 3. On the other hand, the turning mechanism 20 turns the microchip 3 about a rotation axis AX2 (second axis) located at a different position from the rotation axis AX1, then stops the microchip 3, and then turns the microchip 3 about the rotation axis AX1 to produce a centrifugal force in a second direction in the microchip 3.

By controlling the turning mechanism 20 as described above, it is possible to produce a centrifugal force in a first direction in the microchip 3 to separate a component from the specimen in the component separation portion 32 in the microchip 3. It is also possible to produce a centrifugal force in a second direction in the microchip 3 to introduce the component separated in the component separation portion 32 into the reagent reaction portion 33 to make a particular component react with a reagent in the reagent reaction portion 33.

The specimen testing apparatus 1 according to the embodiment includes a housing 2 and a heater 12. The housing 2 houses the microchip 3 and supports the optical detection unit 13. The heater 12 is a temperature control unit that keeps a constant temperature inside the housing 2.

The temperature inside the housing 2 is controlled by the heater 12, and this makes it possible to accurately determine the quantity of a particular component contained in the specimen introduced into the microchip 3 in the housing 2. In particular, since enzyme reactions are temperature-dependent, keeping a constant temperature inside the housing 2 with the heater 12 makes it possible to stably determine the quantity of the particular component based on an enzyme reaction.

The specimen testing method according to the embodiment includes a centrifugal separation step (S3), an introduction step (S5), and a detection step (S7). In the centrifugal separation step, a microchip 3 having a fluid circuit 30 inside it is turned about a first axis (rotation axis AX1) to produce a centrifugal force in a first direction (direction D1) in the microchip 3; thereby a component contained in a specimen introduced into the microchip 3 is centrifugally separated. In the introduction step, the microchip 3 is turned about a second axis (rotation axis AX2) located at a different position from the first axis, is then stopped, and is then rotated about the first axis to produce a centrifugal force in a second direction (direction D2) in the microchip 3; thereby the component separated in the centrifugal separation step is introduced into the reagent reaction portion 33 having a carrier member 330 carrying a reagent. In the detection step, the concentration of the reaction product of part of the component introduced into the reagent reaction portion 33 with the reagent is detected.

With the specimen testing method described above, it is possible to determine the quantity of a particular component based on the detection result in the detection step. Moreover, in the introduction step, by controlling the direction and magnitude of the centrifugal force occurring in the microchip 3, it is possible to control and equalize, for each instance of introduction (partial dispensation) of a component from the component separation portion 32 into the reagent reaction portion 33, the partial dispensation speed and the partial dispensation position. It is thus possible to reduce variations in the reaction time of the particular component with the reagent among instances of partial dispensation. It is thus possible to obtain satisfactory repeatability quantity determination results with the particular component.

### [ 6. Modifications ]

The turning mechanism 20 in the specimen testing apparatus 1 may change the direction of the centrifugal force produced in the microchip 3 and the turning speed of the microchip 3 in accordance with the testing item of the specimen (the type of reagent). In that case, the partial dispensation speed and the partial dispensation position can be changed in accordance with the testing item of the specimen, and thus it is possible, for each testing item, to reliably make the component separated in the component separation portion 32 permeate the carrier member 330 in the reagent reaction portion 33.

While the embodiment deals with a configuration where two stages, namely a first stage 8 and a second stage 9, are provided on the rotary table 6, there may be provided three or more stages on which to place a microchip 3.

The flow rate of the specimen (component) flowing inside the microchip 3 can be controlled at least either from the apparatus or from the chip. From the apparatus it is possible, for example by controlling the direction of the centrifugal force and the turning speed of the microchip 3 with the turning mechanism 20, to control the flow rate of the specimen flowing inside the microchip 3. From the chip it is possible, by appropriately setting the width and length of the micro flow passage constituting the fluid circuit 30, to control the flow rate of the specimen flowing inside the microchip 3.

While the embodiment deals with, as an example, a carrier member 330 that carries a reagent for use in lipid metabolism checks which exhibits a particular enzyme reaction, the carrier member 330 may carry a reagent that exhibits any other enzyme reaction.

While the embodiment deals with, as testing items for the specimen testing apparatus 1, testing items related to lipid metabolism, this is not meant as any limitation. For example, with respect to the liver function, GGT (gamma-GT), ALT, AST, ALP (alkaline phosphatase), T-Bil (total bilirubin), or the like may be taken as a testing item; with respect to the kidney function, Cre (creatinine), UA (uric acid), or the like may be taken as a testing item; with respect to protein in blood, TP (total protein), ALB (albumin), or the like may be taken as a testing item.

A microchip 3 may be configured such that some of the plurality of testing items mentioned above can be measured with a single chip. For example, a microchip 3 can be configured such that GGT, ALT, and AST can be measured with a single chip; or a microchip 3 can be configured such that GGT, AST, and ALP can be measured with a single chip. It is then possible to test the liver function with a single chip.

By measuring Cre and UA with a single chip and measuring CysC by light absorbance measurement, it is possible to test the kidney function. By measuring TP and ALB with a single chip and measuring CRP by light absorbance measurement, it is possible to test protein in blood.

The carrier member described in connection with the embodiment can be any that carries a reagent by, for example, an ion bond, covalent bond, coordinate bond, hydrogen bond, intermolecular bond, metal bond, or the like. Examples of such carriers include activated charcoal for use as a carrier, titania, alumina, silica, anthracite for use as a biofilm filtration material, particulate ceramics, polyethylene, polyurethane, columns for use in various kinds of chromatography, and those employing metal catalysts. Any of these carriers can be prepared in a form with consideration given to reaction efficiency, such as a membrane member, porous member, particulate member, gel-like member, or the like.

The embodiment of the present invention described above is not meant to limit the scope of the present invention, which can be implemented with any modifications and extensions made without departure from the spirit of the invention.

### Industrial Applicability

Microchips according to the present invention can be used in specimen testing apparatuses for testing specimens.

### Reference Signs List

- 1: specimen testing apparatus
- 2: housing
- 3: microchip
- 5: measuring unit
- 12: heater (temperature control unit)
- 13: optical detection unit
- 20: turning mechanism
- 30: fluid circuit
- 31: specimen introduction portion
- 32: component separation portion
- 321: first separation portion (individual separation portion)
- 322: second separation portion (individual separation portion)
- 323: third separation portion (individual separation portion)
- 33: reagent reaction portion
- 330: carrier member
- 330a: development layer
- 330b: light shield layer
- 330c: reaction layer
- 331: first reaction portion (individual reaction portion)
- 332: second reaction portion (individual reaction portion)
- 333: third reaction portion (individual reaction portion)
- 34: waste fluid collection portion

## Claims

1. A microchip having a fluid circuit therein, wherein
the fluid circuit includes:
a specimen introduction portion into which a specimen is introduced;
a component separation portion that, when a centrifugal force in a first direction occurs in the microchip, separates, under the centrifugal force in the first direction, a component contained in the specimen introduced into the specimen introduction portion; and
a reagent reaction portion having a carrier member carrying a reagent, the reagent reaction portion making part of the component introduced from the component separation portion into the carrier member react with the reagent,
wherein
when a centrifugal force in a second direction different from the first direction occurs in the microchip, the component separated in the component separation portion is introduced, under the centrifugal force in the second direction, from the component separation portion into the carrier member.

2. The microchip according to claim 1, wherein
the component separation portion has a plurality of individual separation portions that connect to the specimen introduction portion, and
the individual separation portions each separate and measure, under the centrifugal force in the first direction, the component contained in the specimen introduced into the specimen introduction portion.

3. The microchip according to claim 2, wherein
the reagent reaction portion has a plurality of individual reaction portions that connect to the plurality of individual separation portions, and
the plurality of individual reaction portions each have the carrier member and each make part of the component introduced from one of the individual separation portions into the carrier member under the centrifugal force in the second direction react with the reagent carried by the carrier member.

4. The microchip according to any one of claims 1 to 3, wherein
the carrier member includes:
a development layer in which the component introduced from the component separation portion is diffused and developed; and
a reaction layer located above the development layer and holding the reagent, the reaction layer being permeated by the component developed in the development layer.

5. The microchip according to claim 4, wherein
the carrier member further includes:
a light shield layer located between the development layer and the reaction layer.

6. The microchip according to any one of claims 1 to 5, wherein
the reagent is a dry reagent.

7. The microchip according to any one of claims 1 to 6, wherein
the reagent contains an enzyme.

8. The microchip according to any one of claims 1 to 7, wherein
the fluid circuit further includes:
a waste fluid collection portion that connects to the component separation portion and the reagent reaction portion,
wherein
the waste fluid collection portion is located downstream of the component separation portion in the first direction, and is located downstream of the reagent reaction portion in the second direction.

9. A specimen testing apparatus, comprising:
a rotation mechanism that turns the microchip according to any one of claims 1 to 8; and
an optical detection unit that shines light on the reagent reaction portion of the microchip to receive light reflected therefrom.

10. The specimen testing apparatus according to claim 9, further comprising:
a measuring unit that measures light absorbance of a reaction product.

11. The specimen testing apparatus according to claim 9 or 10, wherein
the rotation mechanism turns the microchip about a first axis to produce the centrifugal force in the first direction in the microchip, and
the rotation mechanism turns the microchip about a second axis located at a different position from the first axis, then stops the microchip, and then turns the microchip about the first axis and thereby produce the centrifugal force in the second direction.

12. The specimen testing apparatus according to any one of claims 9 to 11, further comprising:
a housing that houses the microchip and that supports the optical detection unit; and
a temperature control unit that keeps a constant temperature inside the housing.

13. A specimen testing method, comprising:
a centrifugal separation step of turning a microchip having a fluid circuit therein about a first axis to produce a centrifugal force in a first direction in the microchip, thereby to centrifugally separate a component contained in the specimen introduced into the microchip;
an introduction step of turning the microchip about a second axis located at a different position from the first axis, then stopping the microchip, and then turning the microchip about the first axis to produce a centrifugal force in a second direction in the microchip, thereby to introduce the component separated in the centrifugal separation step into a reagent reaction portion having a carrier member carrying a reagent; and
a detection step of detecting a concentration of a reaction product of part of the component introduced into the reagent reaction portion with the reagent.
